(11) **EP 2 294 254 B1**

(12) **EUROPEAN PATENT SPECIFICATION**

(45) Date of publication and mention
of the grant of the patent:
**05.12.2012 Bulletin 2012/49**

(51) Int Cl.:
*A61B 17/06* (2006.01)  *D01D 5/08* (2006.01)
*D01F 6/04* (2006.01)  *D01F 6/30* (2006.01)

(21) Application number: **09765893.4**

(22) Date of filing: **18.06.2009**

(86) International application number:
**PCT/EP2009/057607**

(87) International publication number:
**WO 2009/153314 (23.12.2009 Gazette 2009/52)**

(54) **ULTRAHIGH MOLECULAR WEIGHT POLYETHYLENE YARN**

GARN AUS ULTRAHOCHMOLEKULAREM POLYETHYLEN

FIL DE POLYÉTHYLÈNE DE MASSE MOLÉCULAIRE TRÈS ÉLEVÉE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **20.06.2008 EP 08158697**

(43) Date of publication of application:
**16.03.2011 Bulletin 2011/11**

(73) Proprietor: **DSM IP Assets B.V.**
**6411 TE Heerlen (NL)**

(72) Inventors:
• **VAZ, Claudia Maria**
**NL-6221 KS Maastricht (NL)**

• **BECKER, Erik**
**NL-6221 CA Maastricht (NL)**
• **WERFF van der, Harm**
**NL-6241 ED Bunde (NL)**

(74) Representative: **Hansen, Jesper Römer**
**DSM Intellectual Property**
**P.O. Box 4**
**6100 AA Echt (NL)**

(56) References cited:
**EP-A- 1 746 187        WO-A-01/73173**
**WO-A-2005/066401      WO-A-2008/116613**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD OF THE INVENTION

The invention relates to an ultrahigh molecular weight polyethylene (UHMwPE) monofilament and to a method for producing thereof. The invention also relates to a medical device comprising the monofilaments.

BACKGROUND OF THE INVENTION

UHMwPE yarn has been described in various publications, including EP 0205960 A, EP0213208A1, US4413110, WO 01 /73173 AI, and Advanced Fiber Spinning Technology, Ed. T. Nakajima, Woodhead Publ. Ltd (1994), ISBN 1-855-73182-7, and references cited therein. In these publications, UHMwPE multifilaments yarns are made by a gel spinning process. Gel spun UHMWPE multifilament yarns have favorable mechanical properties, like a high modulus and a high tensile strength. The gel spun filaments which form the yarns however have the drawback that they contain residual spin solvent. Although the level of residual spin solvent normally is low, it is typically still too high to make the filament suitable for use in medical applications like sutures and cables, since the residual spin solvent may cause unwanted reactions of the human or animal body, like for instance inflammation. Residual spin solvent thus needs to eliminated, e.g. by a cleaning process which may relate to evaporation, extraction or a combination of evaporation and extraction. Such a cleaning process is rather cumbersome, and typically the mechanical properties of the yarns deteriorate during the cleaning process. Furthermore the smoothness of the surface of the filament may be adversely affected during the cleaning process, so that the coefficient of friction of the filament increases. This makes it for example more difficult to stitch a wound with a suture comprising the filament. Also, the rate at which the yarn creeps will increase.

Multifilament yarns have another drawback, in that the small void areas or interstitial spaces between the filaments of multifilament yarns may act as harbor for infectious matters such as bacteria.

EP0740002A1 discloses a yarn made by fusing gel spun polyolefin filaments having a smaller diameter. A yarn is made by this process having a sufficient diameter for fishing lines, with some characteristics of a monofilament. However, this process may potentially lead to internal pores or open pores which may harbor bacteria similar to multifilament yarns. Also, the fused multifilaments show a relatively high surface roughness, making them less suitable for medical applications.

WO 2008/116613 concerns a process for removing spin solvent from a gel spun UHMwPE filament having an effective diameter above 16$\mu$m.

OBJECT OF THE INVENTION

It is an object of the present invention to provide an improved UHMwPE yarn. The improvement may for example be to overcome one or more of the above limitations.

DISCLOSURE OF THE INVENTION

Therefore, a UHMwPE monofilament is provided having a diameter of 30 $\mu$m or more and a spin solvent residue of less than 100 ppm, wherein the monofilament is a gel-spun monofilament, the UHMwPE has an intrinsic viscosity of more than 5 dl/g and the monofilament has a surface roughness Ra of less than 300 nm.

The monofilament has a diameter large enough for use as a yarn in medical applications, e.g. as a suture, from handling perspective and mechanical properties. The monofilament thus does not need to be twisted to make a yarn as in multifilaments, hence reducing the required number of steps and providing a simplified method of making a yarn. Furthermore, the closed structure of the monofilament has no space for attracting bacteria.

For the purposes of the present invention, a yarn is herein understood to mean a product or an article the length dimension of which is much greater than its transverse diameter that can be used as an end-product or for making various other articles. Therefore a yarn herein includes both a yarn made of a plurality of monofilaments and a yarn made of a single monofilament.

A monofilament is herein understood to mean a filament obtainable from a single spin hole. It is noted that a monofilament herein does not include a fused multifilament yarn having some monofilament characteristics, such as the one described in EP0740002A1. For the purposes of the present invention, a monofilament is an elongated body the length dimension of which is much greater than its transverse diameter. Preferably, the monofilaments have a substantially circular or elliptical cross-section. In comparison to the yarn which is a monofilament, a multifilament yarn is herein understood as an elongated body comprising a plurality of individual monofilaments which are arranged to make up a single yarn. The multifilament yarn may potentially include partially fused filaments.

UHMwPE is herein defined as a polyethylene having an intrinsic viscosity (IV) of more than 5 dl/g. Intrinsic viscosity is a measure for molar mass (also called molecular weight) that can more easily be determined than actual molar mass parameters such as Mn and Mw. The IV is determined according to method PTC-179 (Hercules Inc. Rev. Apr. 29,1982) at 135 °C in decalin, the dissolution time being 16 hours, with DBPC as the anti-oxidant in an amount of 2g/l solution, and the viscosity at different concentrations is extrapolated to zero concentration. Because of their long molecule chains, stretched polyolefin fibers with an IV of more than 5 dl/g have very good mechanical properties, such as a high tensile strength, modulus, and energy absorption at break. More preferably, a polyethylene with an IV of more than 10 dl/g is chosen. This is because such gel-spun UHMwPE yarn offers a combination of high strength, low relative density, good hydrolysis resistance, and excellent wear properties, making it particularly suited for use in various biomedical applications, including implants.

Preferably, the UHMwPE of the present invention is a linear polyethylene, i.e. a polyethylene with less than one side chain or branch per 100 carbon atoms, and preferably less than one side chain per 300 carbon atoms, a branch generally containing at least 10 carbon atoms. Preferably, only polyethylene is present, but alternatively the polyethylene may further contain up to 5 mol% of alkenes that may or may not be copolymerized with it, such as propylene, butene, pentene, 4-methylpentene or octene. The polyethylene may further contain additives that are customary for such fibres, such as anti-oxidants, thermal stabilizers, colorants, etc., up to 15 weight %, preferably 1-10 weight %. The UHMwPE may further be added with a polyethylene with lower molecular weight, preferably up to 10 mol%.

The diameter of a monofilament is herein understood to mean the average diameter D of the monofilament calculated from the dtex (g/10km) of the monofilament according to formula 1:

$$D \ (\mu m) = (4/\pi \cdot \rho^{-1} \cdot dtex \cdot 10^{-7})^{1/2} \cdot 10^{6} \qquad \text{Formula 1}$$

wherein density p of the monofilament is assumed to be 970 kg/m$^3$.

The monofilament according to the present invention has a diameter which is large enough to be used as a suture. Filaments having a high diameter are more robust during handling (for example with regard to friction) by a surgeon and more abrasion resistant. Sutures are sized by the USP (United States Pharmacopoeia) scale. A monofilament having a diameter of 30 $\mu$m can be used as a suture of USP 9-0. In another embodiment, the monofilament has a diameter of 40 $\mu$m, which can be used as a suture of USP 8-0. A monofilament having a diameter of 50 $\mu$m may be used as a suture of USP 7-0. The higher diameter provides a higher total strength, although typically the specific strength decreases with a diameter increase. The diameter of the monofilament is preferably not higher than 150 $\mu$m, since it is difficult to eliminate the residual solvent to the level of 100 ppm or less. Even more preferably, the diameter of the monofilament is not higher than 100 $\mu$m.

The residual spin solvent is herein understood to mean the content of the solvent used in making the monofilament, which is still remaining in the final monofilament. In the process of making the monofilament, any of the known solvents for gel spinning of UHMwPE can be used. Suitable examples of spinning solvents include aliphatic and alicyclic hydrocarbons, e.g. octane, nonane, decane and paraffins, including isomers thereof; petroleum fractions; mineral oil; kerosene; aromatic hydrocarbons, e.g. toluene, xylene, and naphthalene, including hydrogenated derivatives thereof, e.g. decalin and tetralin; halogenated hydrocarbons, e.g. monochlorobenzene; and cycloalkanes or cycloalkenes, e.g. careen, fluorine, camphene, menthane, dipentene, naphthalene, acenaphtalene, methylcyclopentandien, tricyclodecane, 1,2,4,5-tetramethyl-1,4-cyclohexadiene, fluorenone, naphtindane, tetramethyl-p-benzodiquinone, ethylfuorene, fluoranthene and naphthenone. Also combinations of the above-enumerated spinning solvents may be used for gel spinning of UHMWPE, the combination of solvents being also referred to for simplicity as spinning solvent. In one embodiment, the spinning solvent of choice has a low vapor pressure at room temperature, e.g. paraffin oil. It was also found that the process of the invention is especially advantageous for relatively volatile spinning solvents at room temperature, as for example decalin, tetralin and kerosene grades. Most preferably, the spinning solvent is decalin.

The combination of the large diameter and the low spin solvent residue makes the monofilament highly suitable for use in medical applications.

The diameter of 30 $\mu$m or more allows the monofilament to be used as a yarn without further twisting or fusing process, with an advantage that there is less possibility of bacteria harboring in pores.

The residual spin solvent content of 100 ppm or less makes the cumbersome cleaning process unnecessary for use in most medical applications. Preferably, the residual solvent content is 80 ppm or less and even more preferably, 60 ppm or less. The lower solvent content makes the monofilament even more suitable for some special medical applications.

In one embodiment of the present invention, the monofilament has a tenacity of 15 cN/dtex or more. Such tenacity is suitable for use in a mesh. In applications where especially high tenacity is required, such as a suture, the monofilament preferably has a tenacity of 25cN/dtex or more.

In one embodiment of the present invention, the surface roughness Ra of the monofilament is 300 nm or less, preferably 250 nm or less. The low surface roughness has an advantage that when used for medical applications such as a suture, a smooth stitching is obtained. The monofilament preferably has a low coefficient of variation of its linear density. The advantage is that it is more homogeneous, i.e. the monofilament shows less differentiation along its length in its mechanical and physical properties. The monofilament has also improved mechanical and physical properties. Moreover, the monofilament shows improved handling during manufacturing or use, especially at elevated speeds as for example in coating processes or in processes including monofilament winding and/or high speed monofilament transportation. a) In another aspect of the present invention, a process for making a UHMwPE monofilament according to claim 7.

It is a general prejudice in the art that filaments having a large diameter will have a problem in that removal of solvents by extraction or evaporation becomes increasingly difficult with increasing diameter. For example, EP0740002A1 states that thicker gel spun filaments would hinder the efficiency and completeness of the solvent removal. In EP0740002A1, this problem is solved by making a yarn with a higher diameter by fusing filaments of a smaller diameter. However, this process requires a step of fusing fibers in addition to the process of making the polyolefin filaments.

On the other hand, according to the present process, it was surprisingly found that the drying of the gel filament before drawing results in a residual solvent of less than 100 ppm in the final monofilament. It was also surprisingly found that drying the gel filament has no substantial affect on the drawability of the monofilament.

The solution of UHMwPE in a solvent is spun from a spinplate comprising one spin hole or a plurality of spin holes. Preferably, the spinning of the filament is done in a manner in which the flow rate of the solution to be spun is controlled. In one embodiment, the solution of UHMwPE is spun from a spinplate comprising a flow rate control means present before the spin hole. The flow rate control means may be a metering pump. In an embodiment wherein the spin plate comprises a plurality of flow rate control means associated with different spin holes, each of the flow rate control means preferably controls the flow rate from the respective spin holes individually. Alternatively, the plurality of flow rate control means may also control the flow rate from different spin holes in the same manner.

The control of the solution flow rate is especially advantageous in this invention, since the effect of an inconstant flow rate is larger in making a larger diameter filament. A large diameter of the spin hole gives a higher possibility that the filament has a variation in its properties over its diameter. This will result in a more homogeneous monofilament.

The invention also relates to a medical device comprising the monofilament according to the present invention. Such medical devices may be a surgical suture consisting of the monofilament according to the present invention.

Surgical sutures must have an extremely high purity since it is used for stitching wound, which is susceptible to infection. A suture consisting of the monofilament according to the present invention is especially advantageous because of its purity and less risk of attracting bacteria. Monofilaments have a stiff and smooth surface, which combine to reduce entanglement. This is also an advantage during the operation of closing wounds.

Another example is a surgical mesh comprising the monofilament according to the present invention. The purity of the monofilament and less risk of harboring bacteria are also advantageous for the surgical mesh. Moreover, the high flexibility and the light weight of the monofilament makes it especially suitable for use as a mesh.

Another aspect of the present invention provides a use of the monofilament according to the present invention for medical application. Good examples of products for medical application include sutures, mesh and cables, but also endless loop products, bag-like, balloon-like products and other woven and/or knitted products. Good examples of cables include a trauma fixation cable, a sternum closure cable, and a prophylactic or per prosthetic cable, long bone fracture fixation cable, small bone fracture fixation cable. Also tube-like products for e.g. ligament replacement are considered.

The invention will be explained more fully below with reference to the following example.

METHODS

- IV: the Intrinsic Viscosity was determined according to method PTC-179 (Hercules Inc. Rev. Apr. 29, 1982) at 135°C in decalin, the dissolution time being 16 hours, with DBPC as anti-oxidant in an amount of 2 g/l solution, by extrapolating the viscosity as measured at different concentrations to zero concentration.
- Dtex: fibers' linear density (dtex, g/10km) was measured by weighing a piece of fiber of predetermined length (about 18 cm). The dtex of the fiber was calculated by dividing the weight in milligrams by 10.
- Tensile properties: tensile tests were carried out on an Instron Z010 tensile tester equipped with a 1 kN load cell and Instron parabolic fiber grips. Tensile strength was determined from the force at break and the linear density measured on each individual sample. Tensile modulus was determined as the chord modulus between 0.3 and 1.0 % strain. Elongation at break and strain were determined by using a gauge length of 100 mm with a tension of 0.08 N at zero strain. The gauge length incorporated the full fiber length on the parabolic grip sections until the beginning of the flat pneumatical grip sections. Strain rate during tensile testing was 50 mm/min.
- Diameter measurement: the diameter was calculated according to formula 1.

- Solvent content: the solvent content was quantitatively measured via 2-step Multiple Headspace Extraction (MHE) gas chromatography (Agilent 6890N Gaschromatograph with an Alltech column filled with AT-wax and a flame ionization detector). A monofilament specimen was put into a sealed headspace vial (Perkin Elmer headspace sampler Turbomatrix 40). The vial was placed into the desorption unit at a temperature of 135 °C for 30 minutes. The vapor phase was injected into the wax column and the solvent is detected with a flame ionization detector. The vial was depressurized and is heated up a second time to 135 °C for 30 minutes. The vapor phase was injected again and the recording of the chromatogram is repeated. The solvent content was calculated on the basis of the peak areas obtained for the sample in the two measurements and the calibration line.
- Surface roughness: the surface roughness Ra was determined with an optical profilometer (Veeco NT1100). A monofilament specimen was gold-coated before the measurement to prevent unwanted internal reflections. The monofilament specimen was scanned using the VSI-mode (vertical scanning interferometry). After scanning, the profile was corrected by cylinder and tilt correction to adjust to the cylindrical shape of the filament using an operating-software of the optical profilometer. The surface roughness was calculated from the scanned data by the operating-software.

Example 1

A UHMWPE- monofilament was made via a gel spinning process. A solution of 10.5 wt% of UHMwPE of IV 18 dl/g in decaline was spun at about 180°C through a spin plate having one spin hole with a diameter of 2mm into a solution monofilament. The solution monofilament was issued from the spin hole to an air-gap of 25 mm and entered a water bath. The solution monofilament was cooled in a water bath kept at about 20°C, and taken-up at such rate that a draw ratio of 3 was applied to the as-spun filaments in the air-gap. Spin velocity was kept constant at 0.64 m/min. The gel filament was dried in air at room temperature for 24 hours. The gel filaments were subsequently further drawn in two steps in an oil-heated tubular oven with a length of 90 cm. The first drawing step was carried out at 130°C with a draw ratio of 5 by setting the entrance speed at 0.2 m/min and the exit speed at 1 m/min. The second drawing step was carried out at 149°C with a draw ratio of 9.5 by setting the entrance speed at 0.063 m/min and the exit speed at 0.6 m/min.

The diameter, the residual solvent content and the surface roughness Ra of the monofilament were measured according to the methods described hereinabove and the results are presented in Table 1. The monofilament has a solvent content and a surface roughness Ra which are low enough to be used for medical applications.

Table 1

|  | Diameter | Solvent content | Surface roughness Ra |
|---|---|---|---|
|  | ($\mu$m) | (ppm) | (nm) |
| Ex. 1 | 55 | 94 | 224 |

The tensile properties of the monofilament were also measured according to the methods described hereinabove and the results are presented in

Table 2. The strength of the monofilament is also high enough to be used as a suture as well as a surgical mesh.

Table 2

|  | Tenacity | E-modulus | Eab |
|---|---|---|---|
|  | (cN/dTex) | (cN/dTex) | (%) |
| Ex. 1 | 28.5 | 1259 | 2.7 |

**Claims**

1. A UHMwPE monofilament having a diameter of 30 $\mu$m or more and a residual spin solvent of less than 100 ppm, wherein the monofilament is a gel-spun monofilament, the UHMwPE has an intrinsic viscosity of more than 5 dl/g and the monofilament has a surface roughness Ra of less than 300 nm.

2. The UHMwPE monofilament according to claim 1, wherein the monofilament has a tenacity of 15 cN/dtex or more, preferably 25 cN/dtex or more.

3. The UHMwPE monofilament according to claim 1 or 2, wherein the diameter is 40 $\mu$m or more, preferably 50 $\mu$m or more.

4. The UHMwPE monofilament according to any one of claims 1-3, wherein the residual spin solvent is less than 80 ppm, preferably less than 60 ppm.

5. The UHMwPE monofilament according to any one of claims 1-4, wherein the diameter is less than 150 $\mu$m, preferably less than 100 $\mu$m.

6. The UHMwPE monofilament according to any one of claims 1-5, wherein the monofilament has a surface roughness Ra of less than 250 nm.

7. A process for making a UHMwPE monofilament, comprising the steps of:

   a) spinning a solution filament from a solution of UHMwPE in a solvent, where the flow rate of the solution to be spun is controlled;
   b) cooling the solution filament obtained to form a gel filament;
   c) drying the gel filament, during which the solvent is at least partly removed; and
   d) drawing the gel filament in at least one drawing step after the step c) of drying the gel filament, to obtain a monofilament having a diameter of 30 $\mu$m or more and a residual spin solvent residue of less than 100 ppm, and a surface roughness Ra of less than 300 nm, and
   the UHMwPE has an intrinsic viscosity of more than 5 dl/g.

8. The process according to claim 7, wherein the step c) of drying of the gel filament is performed at room temperature.

9. The process according to claim 7 or 8, wherein the solvent is decaline.

10. A suture consisting of the UHMwPE monofilament according to any of claims 1-6.

11. The suture of claim 10, having USP 9-0, USP 8-0 or USP 7-0.

12. A cable consisting of the UHMwPE monofilament according to any of claims 1-6.

13. A surgical mesh comprising the UHMwPE monofilament according to any of claims 1-6.

14. A medical device comprising the UHMwPE monofilament according to any of claims 1-6.


**Patentansprüche**

1. UHMwPE-Monofilament mit einem Durchmesser von 30 $\mu$m und mehr und weniger als 100 ppm an restlichem Spinnlösungsmittel, wobei es sich bei dem Monofilament um ein gelgesponnenes Monofilament handelt und das UHMwPE über eine intrinsische Viskosität von mehr als 5 dl/g und das Monofilament über eine Oberflächenrauheit Ra von weniger als 300 nm verfügt.

2. UHMwPE-Monofilament nach Anspruch 1 mit einer Feinheitsfestigkeit von 15 cN/dtex und mehr, bevorzugt 25 cN/dtex und mehr.

3. UHMwPE-Monofilament nach Anspruch 1 oder 2, bei dem der Durchmesser 40 $\mu$m und mehr, bevorzugt 50 $\mu$m und mehr beträgt.

4. UHMwPE-Monofilament nach einem der Ansprüche 1-3, bei dem das restliche Spinnlösungsmittel weniger als 80 ppm, bevorzugt weniger als 60 ppm beträgt.

5. UHMwPE-Monofilament nach einem der Ansprüche 1-4, bei dem der Durchmesser weniger als 150 $\mu$m, bevorzugt weniger als 100 $\mu$m beträgt.

**6.** UHMwPE-Monofilament nach einem der Ansprüche 1-5, bei dem das Monofilament über eine Oberflächenrauheit Ra von weniger als 250 nm verfügt.

**7.** Verfahren zur Herstellung eines UHMwPE-Monofilaments, umfassend die Schritte:

a) Erspinnen eines Lösungsfilaments aus einer Lösung von UHMwPE in einem Lösungsmittel, wobei die Strömungsgeschwindigkeit der zu verspinnenden Lösung kontrolliert wird,
b) Kühlen des erhaltenen Lösungsfilaments zum Gelfilament,
c) Trocknen des Gelfilaments unter zumindest teilweiser Entfernung des Lösungsmittels und
d) Verstrecken des Gelfilaments in mindestens einer Streckstufe nach dem Trocknen des Gelfilaments gemäß Schritt c) unter Erhalt eines Monofilaments mit einem Durchmesser von 30 $\mu$m und mehr und einem Rest an restlichem Spinnlösungsmittel von weniger als 100 ppm und einer Oberflächenrauheit Ra von weniger als 300 nm, wobei
das UHMwPE über eine intrinsische Viskosität von mehr als 5 dl/g verfügt.

**8.** Verfahren nach Anspruch 7, bei dem das Trocknen des Gelfilaments gemäß Schritt c) bei Raumtemperatur erfolgt.

**9.** Verfahren nach Anspruch 7 oder 8, bei dem es sich bei dem Lösungsmittel um Decalin handelt.

**10.** Chirurgisches Nahtmaterial, bestehend aus dem UHMwPE-Monofilament gemäß einem der Ansprüche 1-6.

**11.** Chirurgisches Nahtmaterial nach Anspruch 10 mit USP 9-0, USP 8-0 oder USP 7-0.

**12.** Kabel, bestehend aus dem UHMwPE-Monofilament gemäß einem der Ansprüche 1-6.

**13.** Chirurgisches Netz, umfassend das UHMwPE-Monofilament gemäß einem der Ansprüche 1-6.

**14.** Medizinprodukt, umfassend das UHMwPE-Monofilament gemäß einem der Ansprüche 1-6.


**Revendications**

**1.** Monofilament d'UHMwPE ayant un diamètre de 30 $\mu$m ou plus et une teneur en solvant de filage résiduel inférieure à 100 ppm, dans lequel le monofilament est un monofilament filé à l'état de gel, l'UHMwPE a une viscosité intrinsèque supérieure à 5 dl/g et le monofilament a une rugosité de surface Ra inférieure à 300 nm.

**2.** Monofilament d'UHMwPE selon la revendication 1, dans lequel le monofilament a une ténacité de 15 cN/dtex ou plus, de préférence de 25 cN/dtex ou plus.

**3.** Monofilament d'UHMwPE selon la revendication 1 ou 2, dans lequel le diamètre est de 40 $\mu$m ou plus, de préférence de 50 $\mu$m ou plus.

**4.** Monofilament d'UHMwPE selon l'une quelconque des revendications 1 à 3, dans lequel la teneur en solvant de filage résiduel est inférieure à 80 ppm, de préférence inférieure à 60 ppm.

**5.** Monofilament d'UHMwPE selon l'une quelconque des revendications 1 à 4, dans lequel le diamètre est inférieur à 150 $\mu$m, de préférence inférieur à 100 $\mu$m.

**6.** Monofilament d'UHMwPE selon l'une quelconque des revendications 1 à 5, dans lequel le monofilament a une rugosité de surface Ra inférieure à 250 nm.

**7.** Procédé de fabrication d'un monofilament d'UHMwPE, comprenant les étapes suivantes :

a) le filage d'un filament de solution à partir d'une solution d'UHMwPE dans un solvant, le débit de la solution à filer étant contrôlé ;
b) le refroidissement du filament de solution obtenu pour former un filament de gel ;
c) le séchage du filament de gel, pendant lequel le solvant est au moins partiellement éliminé ; et
d) l'étirage du filament de gel en au moins une étape d'étirage après l'étape c) de séchage du filament de gel, pour obtenir un monofilament ayant un diamètre de 30 µm ou plus et un résidu de solvant de filage résiduel inférieur à 100 ppm, et une rugosité de surface Ra inférieure à 300 nm,
l'UHMwPE ayant une viscosité intrinsèque supérieure à 5 dl/g.

8. Procédé selon la revendication 7, dans lequel l'étape c) de séchage du filament de gel est réalisée à température ambiante.

9. Procédé selon la revendication 7 ou 8, dans lequel le solvant est la décaline.

10. Suture constituée du monofilament d'UHMwPE selon l'une quelconque des revendications 1 à 6.

11. Suture selon la revendication 10, ayant un USP 9-0, USP 8-0 ou USP 7-0.

12. Câble constitué du monofilament d'UHMwPE selon l'une quelconque des revendications 1 à 6.

13. Maille chirurgicale comprenant le monofilament d'UHMwPE selon l'une quelconque des revendications 1 à 6.

14. Dispositif médical comprenant le monofilament d'UHMwPE selon l'une quelconque des revendications 1 à 6.

## REFERENCES CITED IN THE DESCRIPTION

**Patent documents cited in the description**

- EP 0205960 A
- EP 0213208 A1
- US 4413110 A
- WO 0173173 A1
- EP 0740002 A1
- WO 2008116613 A

**Non-patent literature cited in the description**

- Advanced Fiber Spinning Technology. Woodhead Publ. Ltd, 1994